# EUROPEAN PATENT APPLICATION

(11) **EP 2 418 598 A1**
(43) Date of publication of application: **15.02.2012**
(21) Application number: 11176794.3
(22) Date of filing: 08.08.2011
(51) Int. Cl.: G06F 19/00

(54) **Clinical laboratory test information system and non-transitory storage medium**

(30) Priority: 12.08.2010 JP 2010181061
(71) Applicant: Sysmex Corporation, Kobe-shi, Hyogo 651-0073 (JP)
(72) Inventor: Minato, Yukio, Hyogo, 651-0073 (JP); Soyama, Syuichi, Fukuoka, 812-0011 (JP)
(74) Representative: HOFFMANN EITLE

(57) **Abstract**

A clinical laboratory test information system comprising: a memory configured to store time information regarding a plurality of stages included in a clinical-laboratory-test-relating operation; an operating device; a display unit; and a controller configured to: cause the display unit to display, based on the time information stored in the memory, first information indicating a temporal transition of a required time for each of the plurality of stages, such that each stage is selectable; and if one of the plurality of stages is selected by the operating device indicated in the first information, cause the display unit to display second information indicating a temporal transition of the required time for the selected stage.

Also, a non-transitory storage medium.

## Description

### FIELD OF THE INVENTION

The present invention relates to a clinical laboratory test information system and a non-transitory storage medium that can be used in the clinical laboratory test information system.

### BACKGROUND

Clinical laboratory testing systems are known that include supporting functions for allowing clinical-laboratory-test-relating operations to be efficiently processed in laboratories in hospitals, testing centers, and the like (for example, see Japanese Laid-open Patent Publication No. 2003 -279582). A series of clinical-laboratory-test-relating operation includes a plurality of operation processes such as reception of a test request, arrival of a sample, completion of analysis, and confirmation of data. The clinical laboratory testing system disclosed in Japanese Laid-open Patent Publication No. 2003-279582 includes a function of displaying the number of performances of each operation process in one graph with respect to time zone. This clinical laboratory testing system also includes a function of displaying required times for each operation process in one graph.

In laboratories in hospitals, testing centers, and the like, for efficiently performing clinical-laboratory-test-relating operations, it is desired to precisely understand the states of the clinical-laboratory-test-relating operations.

However, in the clinical laboratory testing system according to Japanese Laid-open Patent Publication No. 2003-279582, the number of performances of each of the plurality of operation processes and the required time for each of the plurality of operation processes are displayed in one graph. Accordingly, even when information of a specific operation process is desired to be confirmed, there is a case where it is difficult to identify the information. Therefore, the state of the clinical-laboratory-test-relating operations may not be precisely understood.

Moreover, only by displaying the number of performances of each operation process with respect to each time zone, it is difficult to understand whether each operation process is being smoothly performed without delay. Moreover, only by displaying the required time for each operation process, it is difficult to understand, for example, how the required time for each operation process changes in one day. Therefore, with the clinical laboratory testing system according to Japanese Laid-open Patent Publication No. 2003-279582, it is difficult to appropriately take measures for improving operations, such as positioning appropriate personnel for each time zone and introducing apparatuses to sites where operations tend to delay.

### SUMMARY OF THE INVENTION

The scope of the present invention is defined solely by the appended claims, and is not affected to any degree by the statements within this summary.
(1) According to a first aspect of the present invention, a clinical laboratory test information system comprising: a memory configured to store time information regarding a plurality of stages included in a clinical-laboratory-test-relating operation; an operating device; a display unit; and a controller configured to: cause the display unit to display, based on the time information stored in the memory, first information indicating a temporal transition of a required time for each of the plurality of stages, such that each stage is selectable; and if one of the plurality of stages is selected by the operating device indicated in the first information, cause the display unit to display second information indicating a temporal transition of the required time for the selected stage.
   According to (1), first information indicating a temporal transition of the required time for each of the plurality of stages is displayed on the display unit such that each stage can be designated, and second information indicating a temporal transition of the required time for the stage designated by the operating device from the plurality of stages is displayed on the display unit. This allows the user to easily confirm only the temporal transition of the required time for a specific stage. Further, since the temporal transition of the required time for a specific stage is displayed, it is possible to easily understand how much time in each period the stage requires. Therefore, it is possible to precisely understand the state of the clinical-laboratory-test-relating operation, and thus, the information displayed on the display unit can be effectively used for realizing efficient operations and for improving business operations.
(2) The clinical laboratory test information system according to (1), wherein the first information is information indicating the required time for each stage with respect to each predetermined period, and the second information is information indicating the required time for the selected stage with respect to each predetermined period.
   According to (2), it is possible to easily understand in which period a certain stage in the clinical-laboratory-test-relating operation takes much time. Therefore, it is possible to take appropriate measures for improving operations which would allow reduction of the required time in the period.
(3) The clinical laboratory test information system according to (2), wherein the first information is a first graph indicating the required time for each stage with respect to each predetermined period, and the second information is a second graph indicating the required time for the selected stage with respect to each predetermined period.
   According to (3), it is possible to easily and visually understand the required time for each stage.
(4) The clinical laboratory test information system according to (3), wherein the controller causes the display unit to display, as the first information, a bar graph stacking the required time for each stage with respect to each predetermined period, such that each stage is selectable.
   According to (3), it is possible to easily understand the entire required time for the plurality of stages in each predetermined period, and also it is possible to easily compare the required times for stages included in one period. Moreover, it is possible to easily designate a specific stage via a bar graph stacking the required time for each stage.
(5) The clinical laboratory test information system according to (3) or (4), wherein if a plurality of stages are selected by the operating device, the controller causes the display unit to display, as the second information, a bar graph stacking the required time for each of the selected stages at each of the predetermined period.
   According to (5), it is possible to easily understand the entire required time for the plurality of stages designated by the operating device.
(6) The clinical laboratory test information system according to (5), wherein the memory stores a combination of a plurality of stages, and the controller causes the display unit to show a selecting part which indicates the combination, and if the selecting part is selected by the operating device, the controller causes the display unit to display a bar graph stacking the required time for each stage contained in the combination.
   According to (6), it is not necessary for the user to designate specific stages one by one, whereby the operativity for the user can be improved.
(7) The clinical laboratory test information system according to (6), wherein the memory stores at least two combinations that include a first combination of a plurality of stages and a second combination of a plurality of stages that are different from the plurality of stages contained in the first combination, and when the display unit is displaying a bar graph stacking the required time for each stage contained in the first combination, responsive to a switching instruction, the controller controls the display unit to show a bar graph stacking the required time for each stage contained in the second combination.
   According to (7), it is possible to further improve the operativity for the user.
(8) The clinical laboratory test information system according to any one of (2) to (7), wherein if any stage in any of a plurality of periods indicated in the first information is selected by the operating device, the controller causes the display unit to display the second information.
   According to (8), only by designating a specific stage in one period, it is possible to cause the display unit to display second information of the specific stage. Accordingly, it is possible to improve the operativity for the user.
(9) The clinical laboratory test information system according to any one of (1) to (8), wherein the required time for each stage is an average required time per sample.
   According to (9), the user can take appropriate measures for improving operations in order to enhance the operation efficiency per sample.
(10) The clinical laboratory test information system according to any one of (1) to (8), wherein the required time for each stage is an average required time per test request.
   According to (10), the user can take appropriate measures for improving operations in order to enhance the operation efficiency per test request.
(11) The clinical laboratory test information system according to (9) or (10), wherein the controller causes the display unit to display information indicating a maximum required time and a minimum required time among required times that have been used in calculation of the average required time.
   According to (11), it is possible to more appropriately understand the state of the clinical-laboratory-test-relating operation.
(12) The clinical laboratory test information system according to any one of (1) to (11), wherein the controller receives a selection of a condition for determining a clinical-laboratory-test-relating operation to be displayed, and causes the display unit to display information indicating a temporal transition of the required time for each stage in the clinical-laboratory-test-relating operation meeting the selected condition.
   According to (12), it is possible to precisely understand the state of the clinical-laboratory-test-relating operation on a desired, refined condition.
(13) The clinical laboratory test information system according to (12), wherein the controller receives, as the condition, a selection of a type of at least one of a testing department, a test item, and a testing apparatus.
   According to (13), it is possible to precisely understand the state of the clinical-laboratory-test-relating operation, on a condition refined to a specific type of testing department, test item, or testing apparatus.
(14) The clinical laboratory test information system according to any one of (1) to (13) further comprising a measurement apparatus configured to measure a sample, wherein the plurality of stages include at least one of a measuring stage of a sample performed by the measurement apparatus and an approving stage performed by a user approving a measurement result obtained from the measurement apparatus.
   According to (14), it is possible to easily understand the required time for each stage during a time period from a reception of a patient at a medical institution to payment performed by the patient. This can be utilized in taking measures for improving operations in the standpoint of patients.
(15) According to a second aspect of the present invention, a non-transitory storage medium which stores programs executable comprehensively by at least one processor, the programs causing the at least one processor to perform a method comprising: storing, in a memory, time information regarding a plurality of stages included in a clinical-laboratory-test-relating operation; causing a display unit to display, based on the time information stored in the memory, first information indicating a temporal transition of a required time for each of the plurality of stages, such that each stage is selectable; receiving a selection of a stage by an operating device from the plurality of stages indicated in the first information; and causing the display unit to display, based on the stored time information, second information indicating a temporal transition of the required time for the stage selected by the operating device.

### BRIEF DESCRIPTION OF THE DRAWINGS

FIG. 1 is a schematic diagram showing an overall structure of a test information system according to an embodiment of the present invention;
FIG. 2 shows a hardware configuration of a server management apparatus;
FIG. 3 shows a graph display screen on which a first graph is displayed;
FIG. 4 shows a graph display screen on which a second graph is displayed;
FIG. 5 shows a graph display screen on which a third graph is displayed;
FIG. 6 shows a display condition setting screen;
FIG. 7 is a flow chart showing a flow of operations performed in a hospital;
FIG. 8 is a flow chart showing a flow of operations performed in a hospital;
FIG. 9 shows a table showing contents of a data table;
FIG. 10 is a flow chart showing a procedure of processes performed by an operation state management program;
FIG. 11 is a flow chart showing a procedure of a graph displaying process;
FIG. 12 shows a group setting screen;
FIG. 13 shows a right-click menu; and
FIG. 14 shows a form of displaying the required time period for a stage of a clinical laboratory testing operation according to another embodiment.

### DETAILED DESCRIPTION OF THE PREFERRED EMBODIMENTS

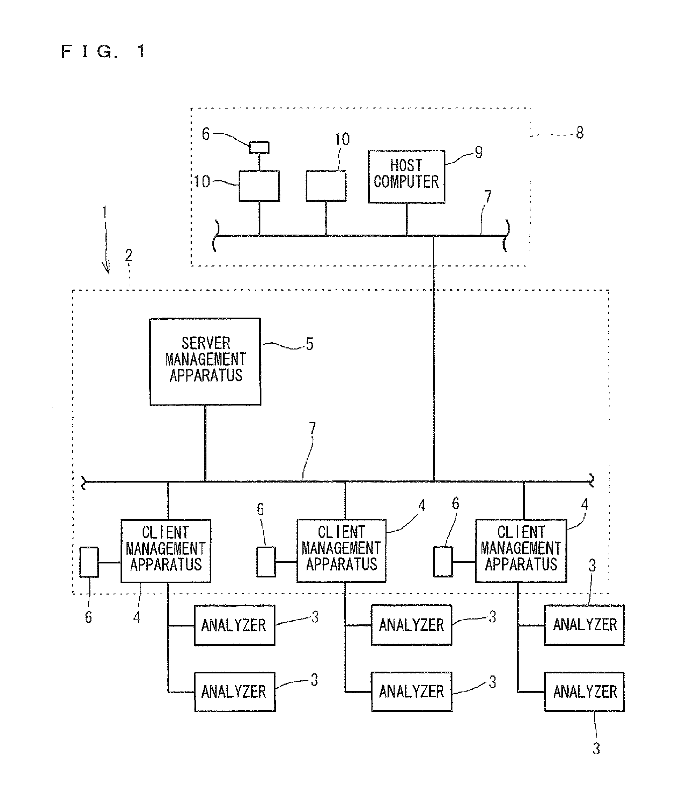

FIG. 1 is a schematic diagram showing an overall structure of a clinical laboratory test information system according to an embodiment of the present invention.

A clinical laboratory test information system 1 according to the present embodiment includes an analyzer 3 placed in a laboratory in a hospital or the like and configured to perform sample analysis, and a management system 2 configured to manage test information, and the analyzer 3. The management system 2 is a client/server-type system including a server management apparatus 5 and a client management apparatus 4. In the example shown in FIG. 1, one server management apparatus 5 and a plurality of (three) client management apparatuses 4 are connected to each other via a communication line 7 in such a manner as to allow data communication.

A plurality of analyzers 3 are connected to each client management apparatus 4. For example, a variety of types of analyzers 3, such as blood cell analyzer, blood coagulation measuring apparatus, biochemical analyzer, immune analyzer, urine component analyzer (urine analyzer), and the like, are connected to a client management apparatus 4. A plurality of analyzers 3 configured to test blood samples are connected to a client management apparatus 4, and a plurality of analyzers 3 configured to test urine samples are connected to another client management apparatus 4. In this manner, a plurality of analyzers 3 using the same type of sample can be connected to one client management apparatus 4.

A label printer (printer) 6 configured to print a collection label to be attached to a sample container (blood collection tube) for containing a sample is connected to a client management apparatus 4. The label printer 6 may be connected to the client management apparatus 4 but is not limited to being so, and may be connected to a network in the test information system 1.

The management system 2 is connected to a host system 8 in a hospital via a communication line. The host system 8 includes a host computer 9, and in addition, a variety of terminals 10, such as order terminals each configured to perform reception and the like of registration of a test order, reception terminals each configured to process reception of a patient's visit to the hospital, payment terminals each configured to perform payment processing, and the like, and these are connected to each other via the communication line 7 in such a manner as to allow data communication. Moreover, the label printers 6 are connected to any of the terminals 10.

When a medical doctor determines that it is necessary to perform a test for a patient, the medical doctor inputs a test request (test order) to an order terminal in the host system 8. The inputted test order is transmitted to the host computer 9 in the host system 8 to be registered. The test order registered in the host computer 9 is further transmitted to the management system 2, to be stored in the server management apparatus 5. Each client management apparatus 4 inquires of the server management apparatus 5 about the test order, and causes analyzers 3 to perform tests based on the test order received from the server management apparatus 5. The server management apparatus 5 receives, from each client management apparatus 4, measurement results obtained from the analyzers 3, and registers the measurement results, formatted in a predetermined reporting form, into the host computer 9 in the host system 8. The report of the measurement results is transmitted from the host computer 9 to the order terminal, to be used by the medical doctor.

### [Hardware configuration of server management apparatus]

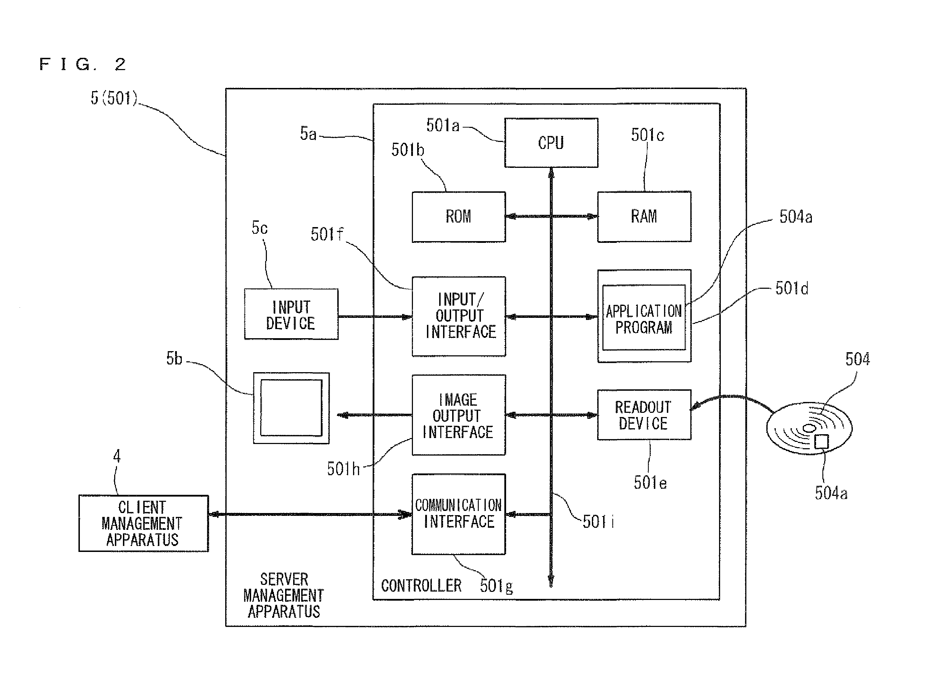

FIG. 2 shows a hardware configuration of the server management apparatus. The server management apparatus 5 is composed of a computer 501 which includes a controller 5a, a display unit 5b, and an input device 5c. It should be noted that the input device 5c may be configured by means of an input device such as a mouse, keyboard, touch panel, and the like. The controller 5a is mainly composed of a CPU 501a; a storage device which includes a ROM 501b, a RAM 501c, a hard disk 501d, and the like; a readout device 501e; an input/output interface 501f; a communication interface 501g; and an image output interface 501h, and these are connected to each other via a bus 501i.

A variety of computer programs, such as an operating system and application programs, to be executed by the CPU 501a, and data to be used for the execution of the computer programs are installed in the hard disk 501 d. In the present embodiment, an application program 504a used for managing the test information and for managing the analyzers 3 is installed in the hard disk 501d. More specifically, an operation state management program to be used for supporting business operation improvement in a hospital, such as realization of efficient testing operations, is installed in the hard disk 501d. Moreover, various types of data to be used in the operation state management program, such as, for example, data regarding performance time of each of the various types of operation stages in a testing operation are stored in the hard disk 50 1d.

As shown in FIG. 2, the input device 5c, such as a keyboard, a mouse, and the like, is connected to the input/output interface 501f. The display unit 5b, such as an LCD, a CRT, or the like, is connected to the image output interface 501h. A display unit of a touch panel type may be used as the display unit 5b.

The communication interface 501g is, for example, Ethernet (registered-trademark) interface. The computer 501 can transmit/receive data to/from other computers (client management apparatuses 4 and host computer 9) by using a predetermined communication protocol such as TCP/IP protocol or the like, by means of the communication interface 501g.

Each client management apparatus 4 is composed of a similar computer to the server management apparatus 5, and therefore, detailed description thereof will be omitted. The label printer 6 and a plurality of analyzers 3 are connected to the input/output interface of the client management apparatus 4.

### [Flow of testing operation in hospital]

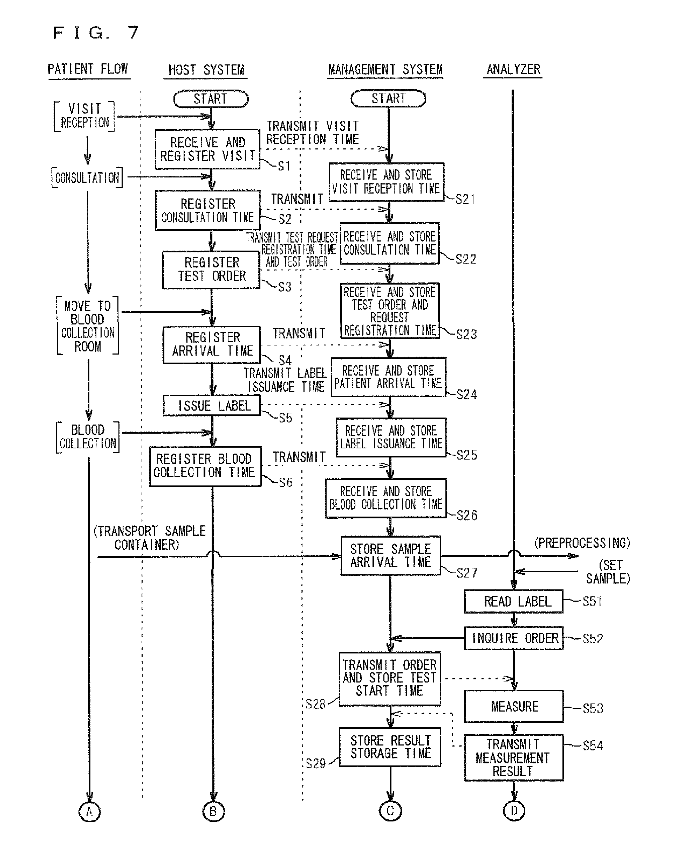

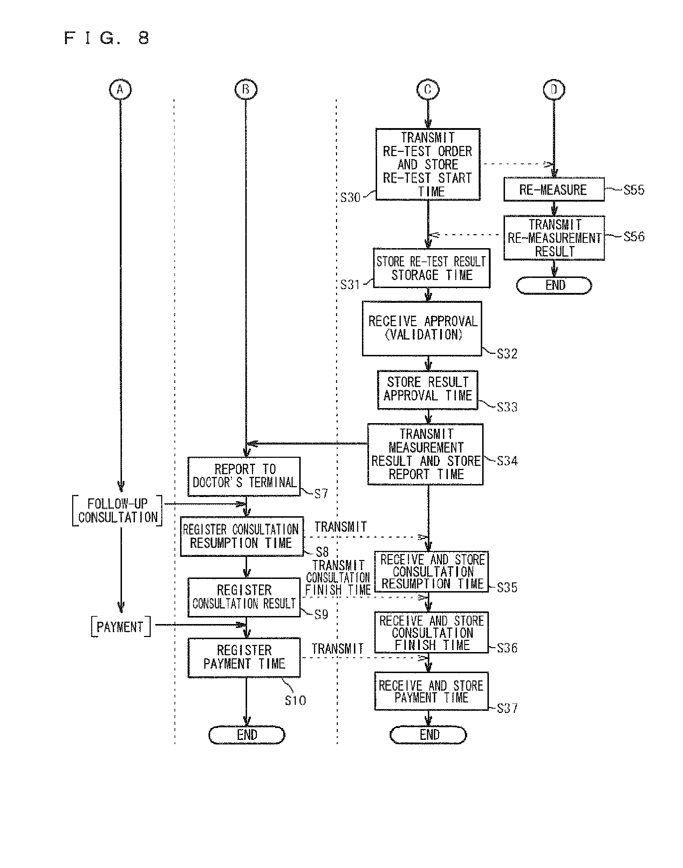

Next, with reference to FIG. 7 and FIG. 8, description will be given of a flow of a testing operation starting from a patient's visit to a hospital as an outpatient, his or her having a consultation and testing, to his or her finishing the payment.

First, a general movement of a patient in a hospital will be described. When a patient visits a hospital, the patient visits the reception counter for reception of the visit, waits for his or her turn, and has a consultation with a medical doctor. If the medical doctor determines that it is necessary to perform a test (here, blood test) at the consultation, the patient receives an instruction to move to a blood collection room and to have his or her blood collected. After the patient has moved to the blood collection room, the patient has his or her blood collected and waits for the test to be finished. After the test is finished, the patient receives, based on the test result, a follow-up consultation with, or treatment by, the medical doctor. Then, after the follow-up consultation is finished, the patient pays.

Next, with reference to FIG. 7 and FIG. 8, description will be given of processes and movements performed on the side of the hospital (the host system 8, the management system 2, and the analyzer 3) in association with the movement of the patient described above.

First, when the patient visits the hospital and finishes the reception at the reception counter, information about the patient (patient ID, etc.) is inputted to a reception terminal of the host system 8 and registered in the host computer 9 (step S1). Moreover, the visit reception time (first time information) is transmitted from the host computer 9 to the server management apparatus 5 in the management system 2 and stored in a memory of the server management apparatus 5 (step S21).

Next, when a consultation with the medical doctor is started, a consultation time is inputted to the order terminal of the medical doctor and transmitted to and registered in the host computer 9 (step S2). Moreover, the consultation time (second time information) is transmitted to the server management apparatus 5 and stored in the memory of the server management apparatus 5 (step S22).

During the consultation, a test request (test order) is inputted to the order terminal by the medical doctor, as necessary, and registered in the host computer 9 (step S3). Then, the test order is transmitted to the server management apparatus 5, along with a request registration time (third time information), and stored in the memory of the server management apparatus 5 (step S23).

After the consultation is finished and the patient arrives at the blood collection room for blood collection, the arrival time is inputted to a terminal in the blood collection room in the host system 8, and transmitted to and registered in the host computer 9 (step S4). Moreover, the arrival time (fourth time information) is transmitted to the server management apparatus 5 and stored in the memory of the server management apparatus 5 (step S24).

Further, based on the test order, a terminal of the host system 8 issues a collection label to be attached to a sample container (step S5). A patient ID, a sample ID (bar code), and the like are printed as information about the sample on this collection label. The time at which the collection label was issued (fifth time information) is transmitted from the host computer 9 to the server management apparatus 5, and stored in the memory thereof (step S25). The collection label may be issued by a printer 6 connected to a client management apparatus 4 in the management system 2. Also in this case, the time at which the collection label was issued is transmitted to the server management apparatus 5 and stored in the memory thereof

When blood of the patient is collected in the sample container with the collection label attached thereto, the time is inputted to a terminal in the blood collection room and transmitted to and registered in the host computer 9 (step S6). Moreover, the collection (blood collection) time (sixth time information) is transmitted from the host computer 9 to the server management apparatus 5, and stored in the memory of the server management apparatus 5 (step S26). The sample container containing the sample is transported to a laboratory, and the time at which the sample container arrived at the laboratory (seventh time information) is inputted to the server management apparatus 5 and stored in the memory thereof (step S27).

In the laboratory, preprocessing of the sample such as centrifugation and the like is performed, as necessary, and then, the sample container is set in an analyzer 3. The analyzer 3 reads the bar code printed on the collection label of the sample container (step S51), and inquires of a corresponding client management apparatus 4 in the management system 2 about a test order for the sample in the sample container (step S52), and further, the client management apparatus 4 inquires of the server management apparatus 5 about the test order. The server management apparatus 5 transmits the test order to the client management apparatus 4, and the client management apparatus 4 transmits the test order to the analyzer 3 and causes the analyzer 3 to perform measurement of the sample. The time is stored as a test start time (eighth time information) in the memory of the server management apparatus 5 (step S28).

The analyzer 3 performs measurement based on the test order (step S53), and transmits the measurement result to the client management apparatus 4 (step S54). Moreover, the client management apparatus 4 transmits the measurement result to the server management apparatus 5. The server management apparatus 5 stores in the memory the time of reception of the test result from the client management apparatus 4, as a result storage time (ninth time information) (step S29).

Based on the measurement result, the client management apparatus 4 determines whether re-testing is necessary, and in a case where re-testing is necessary, the client management apparatus 4 transmits a re-test order to the analyzer 3 and causes the analyzer 3 to perform the re-testing (step S55). The time is transmitted to the server management apparatus 5 as a re-test start time (tenth time information), and stored in the memory of the server management apparatus 5 (step S30).

After re-measurement is finished, the analyzer 3 transmits the re-measurement result to the client management apparatus 4 (step S56), and the client management apparatus 4 transmits the re-measurement result to the server management apparatus 5. The server management apparatus 5 stores in the memory the time of reception of the re-measurement result as a re-test result storage time (eleventh time information) (step S31).

The client management apparatus 4 in the management system 2 receives approval (validation) of the measurement result and the re-measurement result by a laboratory technician (step S32). The time at which the reception of the approval was completed is transmitted from the client management apparatus 4 to the server management apparatus 5 as a result approval time (twelfth time information), and is stored in the memory of the server management apparatus 5 (step S33). Thereafter, the server management apparatus 5 transmits the measurement result, formatted in a predetermined reporting, form to the host computer 9 in the host system 8, and stores the time as a report time (thirteenth time information) in the memory (step S34).

The host computer 9 in the host system 8 transmits the report of the measurement result to the order terminal of the medical doctor (step S7). The medical doctor performs a follow-up consultation for the patient based on the report. The time of the follow-up consultation (resumption of a consultation) is inputted to the order terminal of the medical doctor, and transmitted to and registered in the host computer 9 (step S8). Moreover, the time of the resumption of the consultation (fourteenth time information) is transmitted from the host computer 9 to the server management apparatus 5, and stored in the memory of the server management apparatus 5 (step S35).

After the follow-up consultation is finished, a result of the follow-up consultation is inputted to the order terminal of the medical doctor, and transmitted to and registered in the host computer 9 (step S9). Then, the time at which the result of the follow-up consultation was registered is transmitted from the host computer 9 to the server management apparatus 5 as a consultation finish time (fifteenth time information), and is stored in the memory of the server management apparatus 5 (step S36).

Then, after the patient finishes the payment, information representing the payment having been finished is inputted to a payment terminal in the host system 8, and registered in the host computer 9 (step S10). Moreover, the time at which the payment was finished (sixteenth time information) is transmitted from the host computer 9 to the server management apparatus 5, and stored in the memory of the server management apparatus 5 (step S37).

### [Operation state management by management system]

As described above, the time information of each stage of the testing operation stored in the memory of the server management apparatus 5 in the management system 2 is used for supporting business operation improvement in a hospital, such as realization of efficient operations. Specifically, the management system 2 has a function of displaying in a graph, based on predetermined conditions, the required time period for each stage (this may be also referred to as turnaround time, "TAT") which is calculated by using the performance time of each stage, such that the state of the testing operation is easily understood.

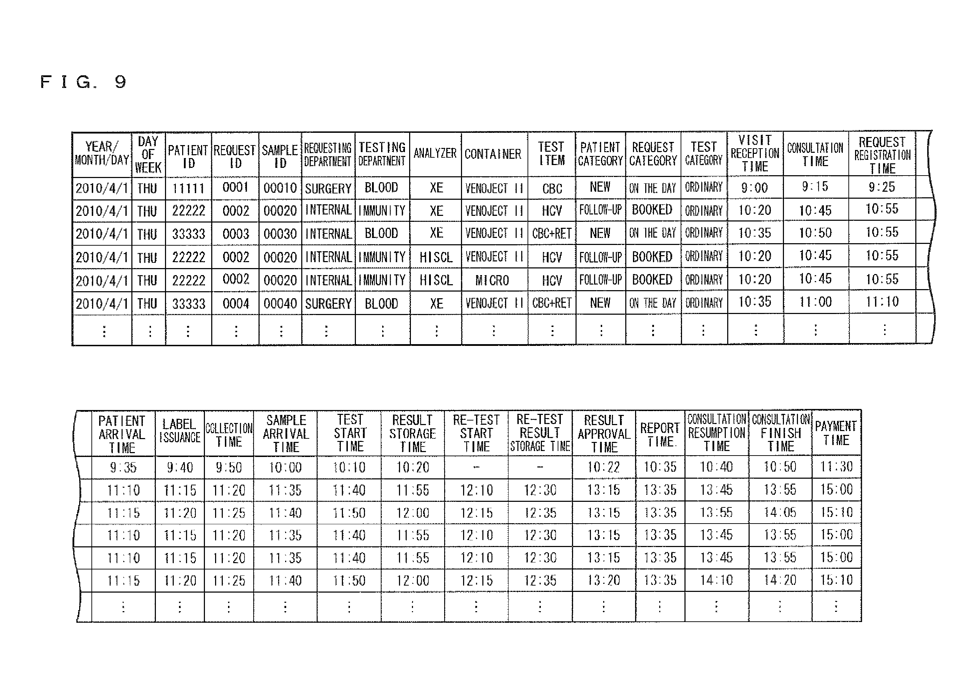

In the memory (the hard disk 501d) of the server management apparatus 5, the above-described types of time information are stored in a data table as shown in FIG. 9. In this data table, each type of the above-described time information is stored along with "year/ month/ day" and "day of week" of the reception of a patient's visit to the hospital, "patient ID", "request ID", "sample ID", "requesting department", "testing department", "analyzer", "container (type)", "test item", "patient category", "request category", and "test category". Further, as described above, in the memory of the server management apparatus 5, the operation state management program is installed for displaying in a graph, by using the data table, the required time period for each stage in the operation performed in the hospital.

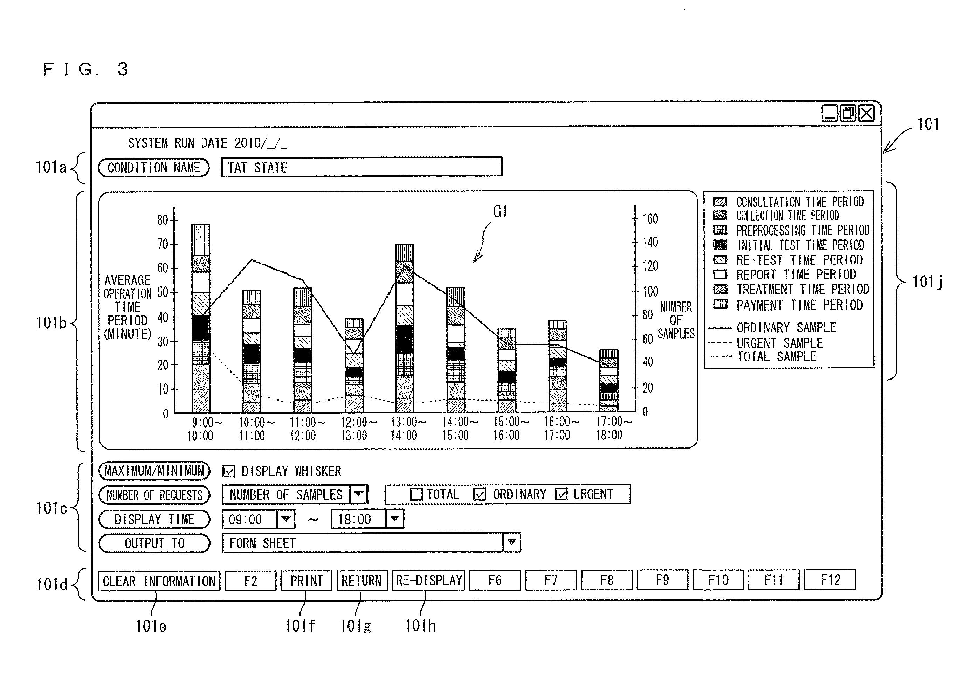

FIG. 3 is a graph display screen 101 showing an example of a graph displayed on the display unit 5b by the operation state management program. The graph display screen 101 includes a title display region 101a for displaying the name of the condition (title) of a graph, a graph display region 101b for displaying the graph, a condition display region 101c for displaying the display condition of the graph, an operation button display region 101d for performing screen operations, and the like.

A graph (first graph G1) displayed on the graph display region 101b is composed of bar graphs, each of which is generated by vertically stacking average operation time periods (required time periods) of respective stages in the testing operation in a corresponding time zone (every hour) during the office hours of a hospital (for example, from 9 a.m. to 6 p.m.). In the present embodiment, the testing operation is categorized in 8 stages, that is, "consultation", "blood collection", "preprocessing", "initial test", "re-test", "report", "treatment", and "payment". The display contents of the respective stages in the bar graph can be confirmed in a legend (display content explanatory region) 101j shown in a right part of the graph display region 101b.

The time period required for performing each stage can be calculated by using the performance time of each operation stored in the data table (see FIG. 9) as follows:
(1) consultation time period: from visit reception time to patient arrival time,
(2) blood collection time period: from patient arrival time to sample arrival time,
(3) preprocessing time period: from sample arrival time to test start time,
(4) initial test time period: from test start time to re-test start time,
(5) re-test time period: from re-test start time to result approval time,
(6) report time period: from result approval time to consultation resumption time,
(7) treatment time period: from consultation resumption time to consultation finish time, and
(8) payment time period: from consultation finish time to payment time.

Further, in addition to the bar graphs, graph G1 shows the number of samples processed in each time zone by means of a line graph. As shown in the legend 101j, the present embodiment is configured such that line graphs can be displayed that indicate the number of ordinary samples, the number of urgent samples, and the number of total samples which is the sum of the number of ordinary samples and the number of urgent samples, respectively.

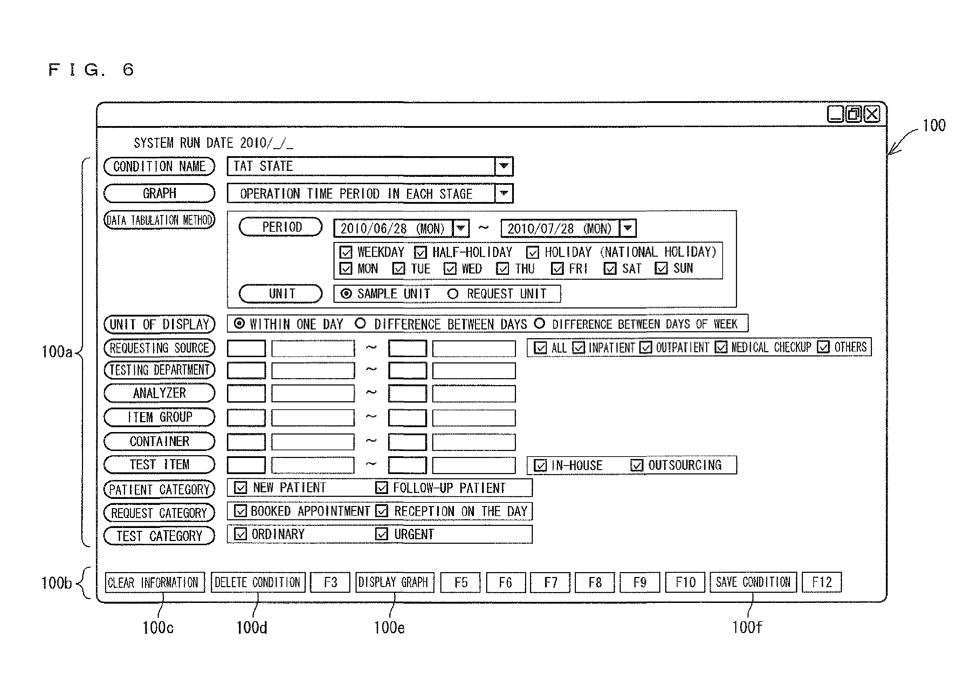

In the condition display region 101c on the graph display screen 101, a part of conditions which are set via a below-described display condition setting screen 100 (see FIG. 6) is displayed, and the condition display region 101c is configured such that the set contents can be changed. Moreover, the operation button display region 101d is provided with an "clear information" button 101e for returning the set contents to their initial states, a "print" button 101f for printing a graph, a "return" button 101g for returning the display to its immediately preceding state, and a "re-display" button 101h for causing a graph to be displayed on a new condition if a change has been made in the condition display region 101c.

First graph G1 is composed of bar graphs, each of which is generated by vertically stacking the required time periods for all of the respective stages in the testing operation. This allows easy understanding of for example, the entire required time period for the stages in each time zone, that is, from a patient visiting the hospital to his or her finishing the payment, and thus, first graph G1 can be utilized in improving the business operations in the standpoint of patients. However, it is difficult to understand from this first graph G1 how much operation time is required for each individual stage, such as consultation or blood collection, in each time zone, and how the operation time period changes.

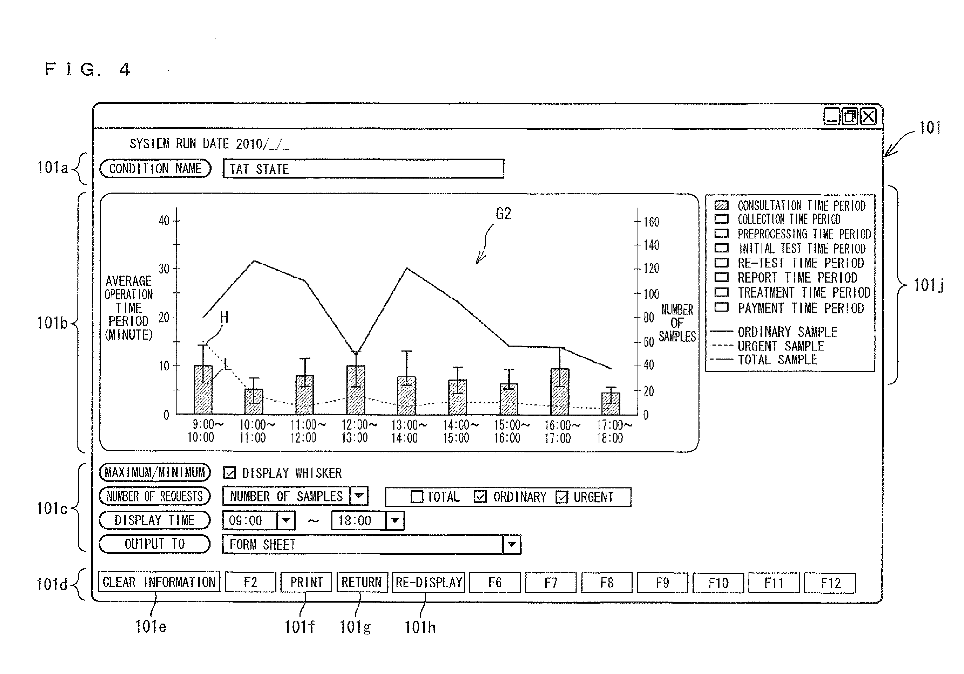

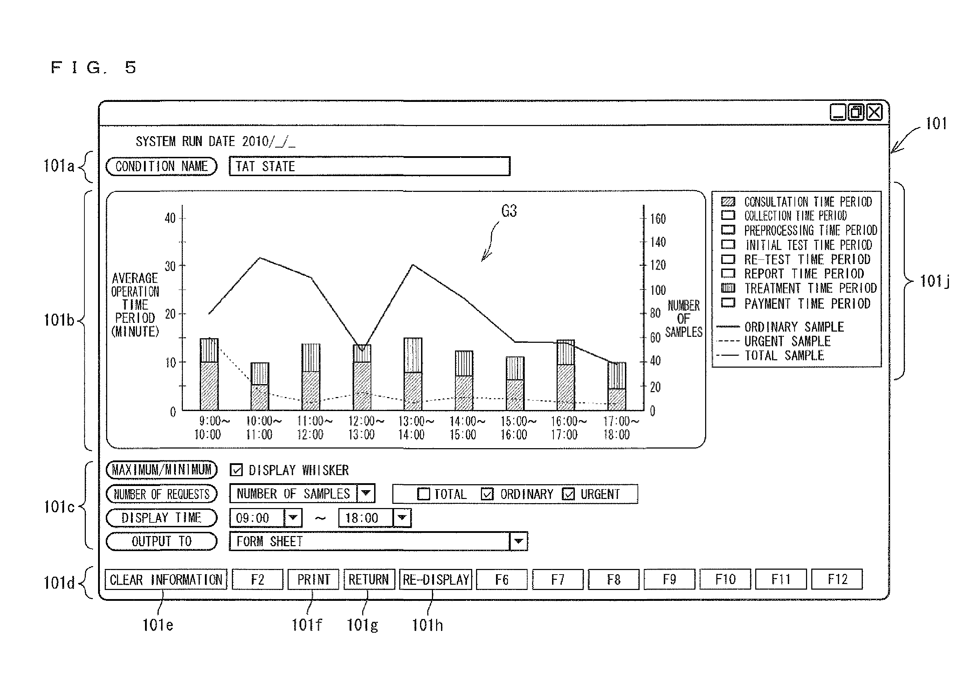

Therefore, the operation state management program according to the present embodiment is configured such that the display unit 5b is allowed to display, not only first graph G1, which is generated by vertically stacking the required time periods for all the respective stages as shown in FIG 3, but also a graph showing the required time period for an individual stage (see second graph G2 in FIG. 4 and third graph G3 in FIG. 5).

As shown in FIG. 4, second graph G2 shows one stage extracted from the stages performed in the testing operation, by means of bar graphs. The horizontal axis represents the time zone (or date, or day of week) and the vertical axis represents the average operation time period (required time period), as in first graph G1. By performing a predetermined operation onto first graph G1, it is possible to display second graph G2.

Specifically, in the state of FIG. 3 in which first graph G1 is being displayed, a portion of a stage among all the stages that constitute a bar graph is left-clicked (designated) by using the input device 5c such as a mouse or the like. Accordingly, second graph G2 is displayed in a state where the designated stage is extracted. Moreover, in the legend 101j, only the item corresponding to the extracted stage is displayed in a conspicuous color, and the other items are displayed (grayed out) in an inconspicuous color such as gray or the like. By indicating, in the legend 101j in such an easily understandable manner, only the item corresponding to the stage that is being displayed in the graph, it is easily understood which stage second graph G2 is showing.

The specific stage to be shown by second graph G2 may be designated via any bar graph of any time zone of first graph G1. Only by designating a specific stage from a bar graph in one time zone, it is possible to cause second graph G2 to show average operation time periods in all the time zones regarding the designated stage. Moreover, the specific stage to be shown by second graph G2 can be designated from the stages shown in the legend 101j .

According to the present embodiment, it is possible to display each bar graph representing the average operation time period of the one extracted stage, along with the maximum value and the minimum value of the operation time periods used in calculating the average operation time period. Specifically, an upper horizontal-line (whisker) H indicating the maximum value and a lower horizontal-line (whisker) L indicating the minimum value are displayed, above and below the top of each bar graph, respectively. In order to display such maximum values and minimum values, it is only necessary to check "display whisker" in the item "maximum/minimum" in the condition display region 101c which is displayed below second graph G2. As described above, by displaying the maximum value and the minimum value along with the average operation time period, it is possible to recognize the degree of variation of the operation time periods. This allows more precise understanding of the state of the testing operation.

Further, according to the present embodiment, as in graph G3 (third graph) shown in FIG. 5, it is possible to display each bar graph representing a plurality of extracted stages, instead of representing only one stage. Specifically, when a plurality of stages are designated from one of the bar graphs in first graph G1, that is, for example, when a plurality of stages are left-clicked with a mouse while the control key of the keyboard is being pressed, a bar graph indicating the average operation time periods of only the extracted plurality of stages is displayed in each time zone.

Displaying third graph G3 indicating the extracted plurality of stages can allow easy understanding of the states of the operations. For example, stages that a medical doctor is mainly involved with include two stages, that is, consultation stage and treatment stage. Therefore, by extracting these two stages and causing third graph G3 to display them, a medical doctor can easily understand at which time zone operations take much time. In particular, in first graph G1, the consultation stage (time period) and the treatment stage (time period) are displayed in a manner where they are vertically spaced away from each other. Therefore, it is very difficult to understand a combined average operation time period of the two stages. However, in third graph G3, it is possible to display the stages in a vertically, directly stacked manner, which stages are displayed away from each other in first graph G1. This allows easy understanding of the total of the average operation time periods of the plurality of stages.

Moreover, in the present embodiment, as a method for causing a graph representing one or a plurality of extracted stages to be displayed as in second graph G2 or third graph G3, it is possible to set in advance groups of stages to be displayed.

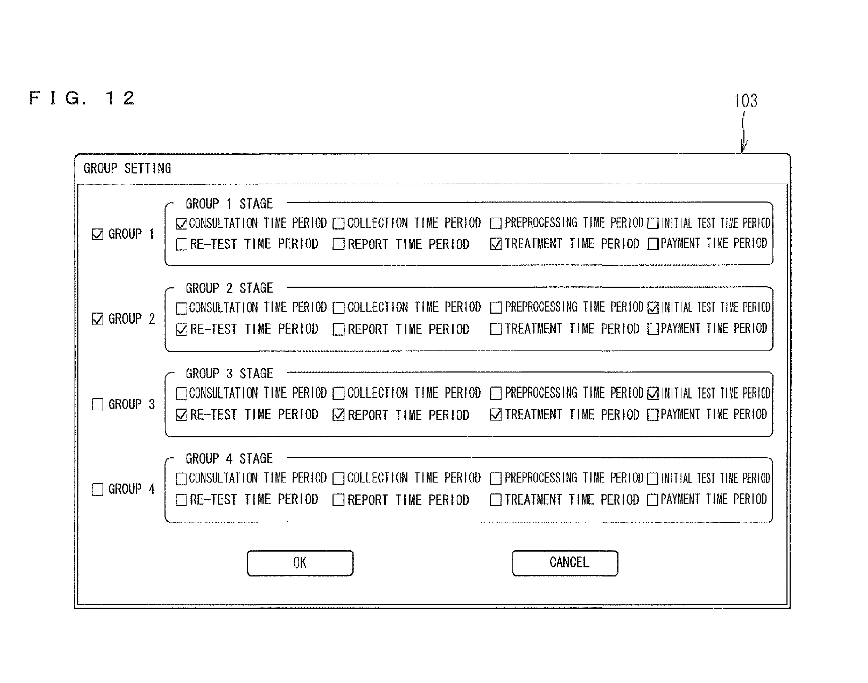

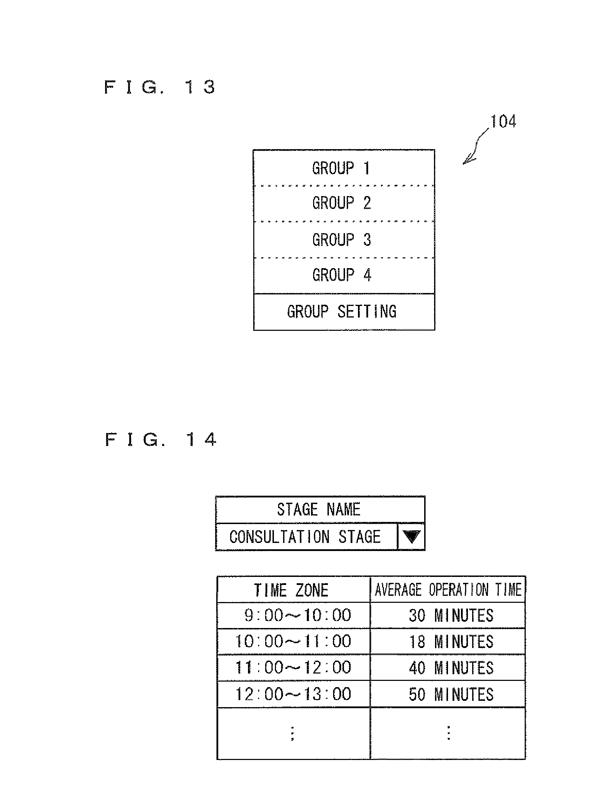

Specifically, by right-clicking first graph G1 shown in FIG 3, a right-click menu (auxiliary operation screen) 104 shown in FIG. 13 is displayed. The right-click menu 104 allows selection of a group from group 1 to group 4 and selection of group setting. If the group setting is selected here, a group setting screen 103 shown in FIG. 12 is displayed.

On the group setting screen 103, any or all of four groups, that is, groups 1 to 4, can be designated via check boxes, and with respect to each of groups 1 to 4, one or more stages that are desired to be displayed in a graph can be designated via check box(es). In the shown example, group 1 and group 2 are designated. With respect to group 1, the consultation time period and the treatment time period concerning the operations by a medical doctor are designated. With respect to group 2, the initial test time period and the re-test time period concerning the operations of analyzers are designated. Thus, by designating desired groups and stages and clicking an OK button, the group setting is performed.

When first graph G1 is right-clicked, only the group(s), among groups 1 to 4, that have been designated in advance in the group setting turn into states that allow selection thereof. Then, when one of the selectable group(s) from among groups 1 to 4 is selected, only the stage(s) designated in that group are displayed in second graph G2 or third graph G3. Further, also when second graph G2 or third graph G3 is right-clicked, the right-click menu 104 is similarly displayed, thereby allowing setting of groups or selecting another group to switch the display.

As described above, instead of designating a plurality of stages one by one from first graph G1, by setting in advance as a group a combination of one or a plurality of stages to be displayed in a graph, it is possible to cause, through fewer operations, second graph G2 or third graph G3 to be displayed which indicates only desired stage(s). Accordingly, it is possible to improve the operativity for the user.

Next, conditions for displaying the above graphs will be described. With the operation state management program according to the present embodiment, it is possible to display the average operation time period of a stage in a graph, with the display condition refined to, for example, a testing operation performed at a specific testing department or a testing operation performed for a specific test item. When the operation state management program is started in the server management apparatus 5 and predetermined operations are performed, the display condition setting screen 100 shown in FIG. 6 is displayed on the display unit 5b.

The display condition setting screen 100 is provided with a setting item display region 100a and an operation button display region 100b. The setting item display region 100a is provided with setting items of "condition name", "graph", "data tabulation method" ("period", "unit"), "unit of display" "requesting source", "testing department", "analyzer", "item group"_{,} "container", "test item", "patient category", and "request category".

The setting item "condition name" allows a user to select one of preset condition names or to input any new condition name. In the shown example, a condition name of "TAT (turn around time) state" is set.

The setting item "graph" allows selection of one of preset types of graph. In the present embodiment, in order to cause the graphs shown in FIG. 3 to FIG. 5 to be displayed, a type of graph called "operation time period in each stage" is set.

The setting item "data tabulation method" allows setting of "period" and "unit". With respect to the setting item "period", the first year/ month/ day and the last year/ month/ day of a data tabulation period that is desired to be displayed in a graph are set. Further, in a case where it is desired to perform a tabulation on a refined condition of a specific day (day of week) within that data tabulation period, it is possible to select one or more of "weekday", "half-holiday", "holiday (national holiday)", and "Monday" through "Sunday".

The setting item "unit" allows selection of either one of "sample unit" or "request unit". "Sample unit" can be selected in a case where the average operation time period per sample is to be displayed in a graph, and "request unit" can be selected in a case where the average operation time period per request (test order) is to be displayed in a graph. For example, in a case where a medical doctor in a medical department has requested a test that requires a plurality of samples for one patient, if "sample unit" is selected, the average operation time periods of the respective operation stages per sample are calculated, and if "request unit" is selected, the average operation time periods of the respective operation stages are calculated with all the samples included in one request totaled. The graph displayed in "sample unit" can be utilized for improving efficiency and the like of operations relating to analyzers and laboratory technicians that handle the samples one by one. The graph displayed in "request unit" can be utilized for business operation improvement and the like for a patient who undergoes tests for each request. In a case where the average operation time period is calculated in "sample unit", the number of samples identified by the sample IDs in the data table shown in FIG. 9 is used. In a case where the average operation time period is calculated in "request unit", the number of requests identified by the request IDs in the data table is used.

The setting item "unit of display" allows selection of a unit of display from three choices of "within one day", "difference between days", and "difference between days of week". Here, if "within one day" is selected, the average operation time period of each stage is displayed, using the time zone as the horizontal axis, as in the graph shown in FIG. 3. If "difference between days" is selected, the average operation time period of each stage is displayed, using the days contained in the data tabulation period set in "period" in "data tabulation method" as the horizontal axis. If "difference between days of week" is selected, the average operation time period of each stage is displayed, using the days of week contained in the data tabulation period set in "period" in "data tabulation method" as the horizontal axis. That is, in the present embodiment, it is possible to display the average operation time periods in a graph, in corresponding time sections (period sections), that is, time zones, days, and days of week. It should be noted that, in addition to these time sections, other time sections such as week and month may be employed.

As other conditions, it is possible to select "requesting source" (selection of a medical department being a requesting source, such as internal medicine, surgery, or the like), "testing department" (selection of the type, such as blood, immunity, or the like), "analyzer" (selection of the type, such as blood, immunity, or the like), "item group", "container (sample container)", "test item", "patient category", "request category", and "test category". With respect to "requesting source" to "test item", the condition that is desired to be refined can be inputted by use of a code or the like. With respect to "test item", the condition can be further refined by means of "in-house" and "outsourcing".

With respect to "patient category", the condition can be refined by means of "new patient" and "follow-up patient". With respect to "request category", the condition can be refined by means of "booked appointment" and "reception on the day". With respect to "test category", the condition can be refined by means of "ordinary" and "urgent". The above setting of conditions can be performed by using information recorded in the data table shown in FIG. 9 and information associated therewith.

Although the present embodiment is provided with the above setting conditions, the setting conditions are not limited thereto. A part of these setting conditions may be allowed to be set, or other setting items may be provided.

When desired conditions are set on the display condition setting screen 100 and a "display graph" button 100e in the operation button display region 100b in a lowest part of the display condition setting screen 100 is clicked (selected) by means of a mouse or the like, graph (first graph) G1 shown in FIG. 3 is displayed with the desired conditions being satisfied. When a "clear information" button 100c in the operation button display region 100b is clicked, the conditions set in the setting item display region 100a return to their initial states. When a "delete condition" button 100d is clicked, the conditions being displayed are deleted. When a "save condition" button 100f is clicked, the set conditions are saved in the memory 5b.

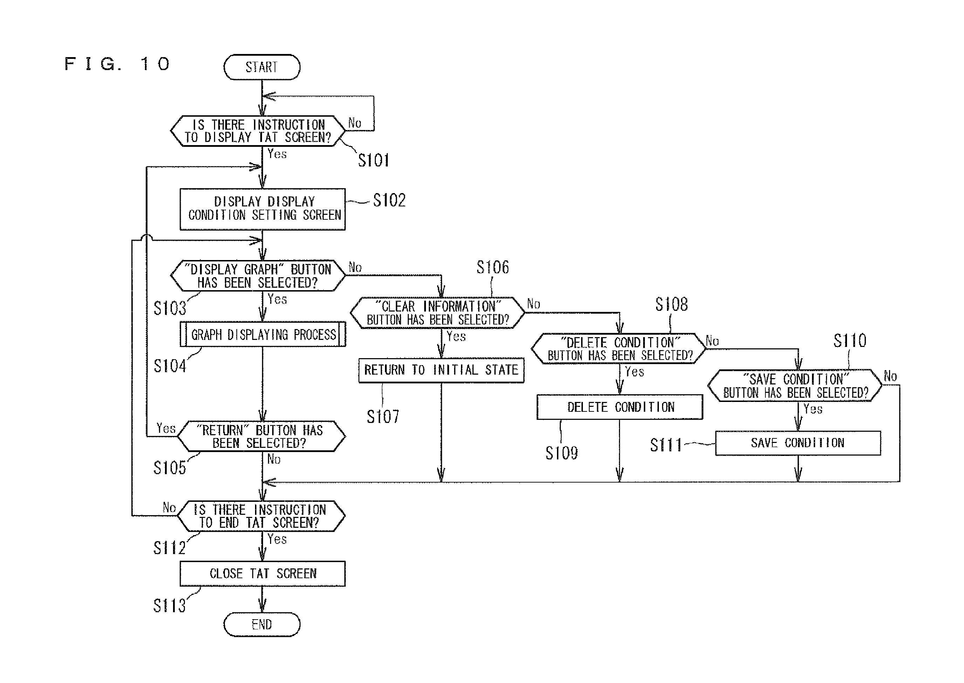

Next, with reference to a flow chart in FIG. 10, description will be given of processing for causing the above described graphs G1 to G3 to be displayed when the operation state management program is executed in the server management apparatus 5.

In step S101 in FIG. 10, the CPU 501a of the server management apparatus 5 determines whether the operation state management program has been executed and an display instruction to display a required time period display screen (TAT screen) of a testing operation has been issued. When the CPU 501a has determined that the display instruction has been issued, the CPU 50 1 a advances the processing to step S102, and causes the display unit 5b to display the display condition setting screen. 100 shown in FIG. 6. A user can set the display condition as described above on the display condition setting screen 100.

Next, in step S103, the CPU 501a determines whether the "display graph" button 100e in the display condition setting screen 100 has been selected. When the CPU 501 a has determined that the "display graph" button 100e has been selected, the CPU 501a performs a graph displaying process in step S104. When the CPU 501a has determined that the "display graph" button 100e has not been selected, the CPU 501 a advances the processing to step S106.

In step S104, when the graph displaying process has been performed, the CPU 501a determines whether the "return" button 101g in the graph display screen 101 (see FIG. 3) has been selected. When the CPU 501a has determined that the "return" button 101g has been selected, the CPU 501a returns the processing to step S102, and causes the display unit 5b to display the display condition setting screen 100 again. When the CPU 501a has determined that the "return" button 101g has not been selected, the CPU 501a advances the processing to step S 112.

In step S106, the CPU 501 a determines whether the "clear information" button 100c in the display condition setting screen 100 has been selected, and when the CPU 501a has determined that the "clear information" button 100c has been selected, the CPU 501a advances the processing to step S107, performs a process of returning the display condition to its initial state, and then advances the processing to step S112. When the CPU 501a has determined that the "clear information" button 100c has not been selected, the CPU 501 a advances the processing to step S108 .

In step S108, the CPU 501a determines whether the "delete condition" button 100d in the display condition setting screen 100 has been selected. When the CPU 501 a has determined that the "delete condition" button 100d has been selected, the CPU 501a advances the processing to step S109, and when the CPU 501 a has determined that the "delete condition" button 100d has not been selected, the CPU 501a advances the processing to step S110.

In step S109, the CPU 501 a deletes the setting of the display condition set on the display condition setting screen 100, and advances the processing to step S 112.

In step S110, the CPU 501a determines whether the "save condition" button 100f in the display condition setting screen 100 has been selected. When the CPU 501 a has determined that the "save condition" button 100f has been selected, the CPU 501a advances the processing to step S111, and when the CPU 501 a has determined that the "save condition" button 100f has not been selected, the CPU 501 a advances the processing to step S 112.

In step S111, the CPU 501a saves the condition set on the display condition setting screen 100 in the hard disk 501 d, and advances the processing to step S112.

In step S112, the CPU 501a determines whether an instruction to end displaying the TAT screen has been issued. When the CPU 501a has determined that the instruction to end displaying the TAT screen has been issued, the CPU 501 a closes the TAT screen and ends the processing, and when the CPU 501a has determined that the instruction to end displaying the TAT screen has not been issued, the CPU 501a returns the processing to step S102.

Next, the graph displaying process in step S104 will be described further in detail.

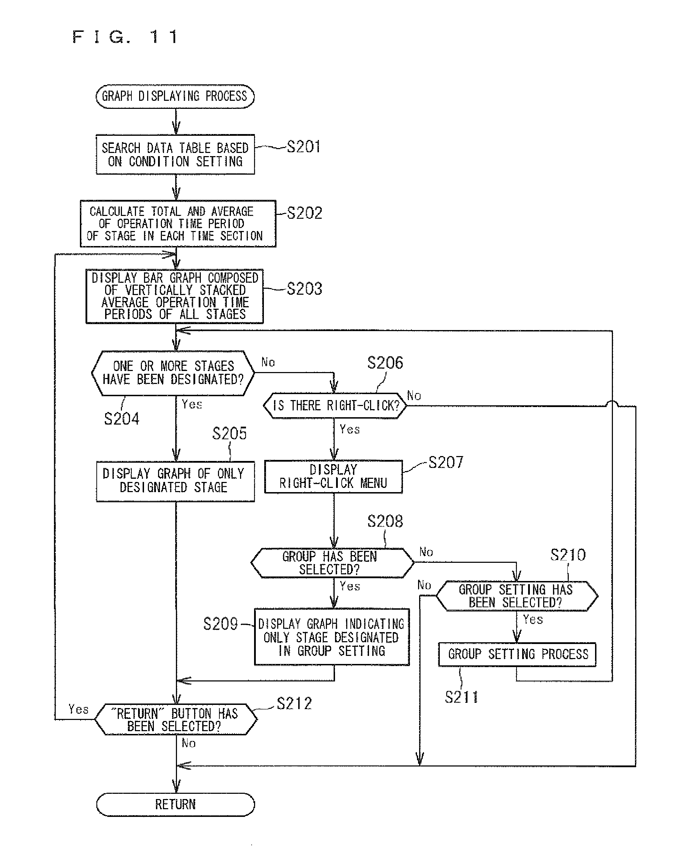

FIG. 11 is a flow chart showing the procedure of the graph displaying process.

In step S201 in FIG. 11, the CPU 501a searches the data table shown in FIG. 9, based on the display condition settings made on the display condition setting screen 100.

Next, in step S202, the CPU 501a calculates the total sum of operation time periods of respective stages in each time zone, and calculates the average operation time period per sample or per request.

Next, in step S203, the CPU 501a causes the display unit 5b to display the graph display screen 101 (see FIG. 3) in which bar graph (first graph) G1 is displayed in the graph display region 101b, each bar graph of bar graph G1 being generated by vertically stacking average operation time periods of all of the respective stages of the testing operation.

Next, in step S204, the CPU 501a determines whether one or a plurality of stages have been designated in first graph G1. This designation is performed by left-clicking one or a plurality of stages in a bar graph in first graph G1, as described above. When the CPU 501 a has determined that one or a plurality of stages have been designated, the CPU 501 a extracts, in step S205, only the designated one or the plurality of stages, causes second graph G2 or third graph G3 to be displayed on the graph display screen 101, and then advances the processing to step S212. When the CPU 501a has determined that one or a plurality of stages have not been designated, the CPU 501a advances the processing to step S206.

In step S206, the CPU 501 a determines whether the graph display screen 101 has been right-clicked. When the CPU 501a has determined that the graph display screen 101 has been right-clicked, the CPU 501a advances the processing to step S207, and when the CPU 501a has determined that the graph display screen 101 has not been right-clicked, the CPU 501 a returns the processing.

In step S207, the CPU 501a causes the display unit 5b to display the right-click menu 104 shown in FIG. 13, and in step S208, the CPU 501a determines whether one of groups 1 to 4 has been selected. When the CPU 501a has determined that one of groups 1 to 4 has been selected, the CPU 501a causes, in step S209, second graph G2 or third graph G3 indicating only the stage(s) designated in the group setting screen 103 shown in FIG. 12, to be displayed on the graph display screen 101. When the CPU 501a has determined that one of groups 1 to 4 has not been selected, the CPU 501a advances the processing to step S210.

In step S210, the CPU 501a determines whether the group setting has been selected from the right-click menu 104. When the CPU 501a has determined that the group setting has been selected, the CPU 501a causes, in step S211, the group setting screen 103 to be displayed, and returns the processing to step S204 after the group setting has been performed. When the CPU 501a has determined that the group setting has not been selected, the CPU 501a returns the processing.

In step S212, the CPU 501a determines whether the "return" button 101g (see FIG. 4 and FIG. 5) in the graph display screen 101 has been selected while second graph G2 or third graph G3 is being displayed. When the CPU 501a has determined that the "return" button 101g has been selected, the CPU 50 1 a returns the processing to step S203, causes bar graph (first graph) G1 to be displayed again, each bar graph of the bar graph G1 being generated by vertically stacking average operation time periods of all of the respective stages. When the CPU 501a has determined that the "return" button 101g has not been selected, the CPU 501a returns the processing.

As described above in detail, according to the present invention, it is possible to cause the temporal transition of the average operation time periods of a plurality of stages of a testing operation to be displayed in first graph G1, and it is possible to extract one or more stages designated from among the displayed stages, and then cause the display unit 5b to display second graph G2 or third graph G3, which shows the temporal transition of the average operation time periods of the extracted one or more stages. Accordingly, a user can easily confirm the temporal transition of the operation time periods of a specific stage only. Further, since the temporal transition of the operation time periods of the specific stage is displayed, the user can easily understand how much operation time is required in a certain period, for the specific stage. For example, the user can easily understand how much operation time is required in each time zone (day, or day of week). Therefore, with respect to a stage that requires much operation time in a certain time zone, it is possible to appropriately take measures for improving business operations that would reduce the operation time period.

As described above in detail, according to the present invention, it is possible to designate one or more desired stages from among all of the stages of the testing operation displayed in first graph G1, and to cause the display unit 5b to display second graph G2 or third graph G3, which indicates the average operation time periods of the extracted one or more designated stages. Therefore, it is possible to easily understand how much operation time is required for a specific stage in each time zone (or day, or day of week). Accordingly, with respect to a stage that requires much operation time in a certain time zone, it is possible to appropriately take measures for improving business operations that would reduce the operation time period.

Moreover, since specific one or more stages can be directly designated from the display of first graph G1, the operativity for the user can be enhanced. Moreover, by designating specific one or more stages from a bar graph in a time zone displayed in first graph G1, it is possible to display second graph G2 or third graph G3 indicating the bar graphs of all of the time zones regarding the designated one or more stages. This can further enhance the operativity for the user.

With respect to first graph G1 to third graph G3, it is possible to refine the condition by selecting, via the display condition setting screen 100 shown in FIG. 6, testing operations to be displayed in a graph. Therefore, the present invention is very useful, for example, in a case where the user desires to understand the states only of the clinical laboratory testing operations performed in a specific testing department, a specific analyzer, or the like.

The present invention is not limited to the above-described embodiment, and can be modified as appropriate without departing from the scope of the invention defined in the claims.

For example, in the above embodiment, the average operation time periods of specific stages of the clinical laboratory testing operation are displayed in the bar graphs. However, they may be displayed in a form of a table as shown in FIG. 14.

Further, in the above embodiment, the average operation time periods per sample or per request are displayed in a corresponding time zone (or day, day of week) in a graph. However, the total time period of the operation time periods used for calculation of the average operation time periods, the medium value in a time zone, and the like, may be displayed in a corresponding time zone. Still further, although the performance time of each stage is stored in the hard disk 501 d of the server management apparatus 5, an average operation time period calculated using the performance time may be stored therein.

Further, in the above embodiment, the average operation time period of each stage is displayed in a corresponding time zone (or day, or day of week) in a form of a bar graph. However, any display manner may be employed only if the temporal transition of the average operation time period of each stage is known. For example, a line graph indicating the temporal transition of the average operation time period of each stage may be displayed.

Further, the storing manner of the performance time of each operation performed in a clinical laboratory testing operation is not limited to that in which the server management apparatus 5 receives and stores each performance time transmitted from the host computer 9 or the client management apparatus 4. Alternatively, the user may directly input the performance time by using the input device 5c such as a keyboard.

In the above embodiment, the operation state management program is installed in the server management apparatus 5, and the display unit 5b of the server management apparatus 5 displays a graph of the required time period for each stage performed in a clinical laboratory testing operation. However, a similar operation state management program may be installed in a client management apparatus 4 and the graph may be displayed on the display unit of the client management apparatus 4. In this case, the client management apparatus 4 may receive from the server management apparatus 5 the performance time and the like of each stage recorded in the data table, and may calculate the average operation time period of each stage.

Further, in the above embodiment, when the server management apparatus 5 receives a start time of each operation (stage), the server management apparatus 5 stores the start time of each stage in the data table (see FIG. 9), and when the server management apparatus 5 receives an instruction to display a graph, the server management apparatus 5 calculates the time period required for performing each stage based on the start time, and causes the graph to be displayed. However, the present invention is not limited thereto. For example, the server management apparatus 5 receives a start time of each stage, calculates the time period required for performing the stage in advance based on the received start time, and stores the calculated time in the data table. Then, when the server management apparatus 5 receives an instruction to display a graph, the server management apparatus 5 may cause the graph to be displayed based on the calculated time period.

## Claims

1. A clinical laboratory test information system comprising:
a memory configured to store time information regarding a plurality of stages included in a clinical-laboratory-test-relating operation;
an operating device;
a display unit; and
a controller configured to:
cause the display unit to display, based on the time information stored in the memory, first information indicating a temporal transition of a required time for each of the plurality of stages, such that each stage is selectable; and
if one of the plurality of stages is selected by the operating device indicated in the first information, cause the display unit to display second information indicating a temporal transition of the required time for the selected stage.

2. The clinical laboratory test information system according to claim 1, wherein
the first information is information indicating the required time for each stage with respect to each predetermined period, and
the second information is information indicating the required time for the selected stage with respect to each predetermined period.

3. The clinical laboratory test information system according to claim 2. wherein
the first information is a first graph indicating the required time for each stage with respect to each predetermined period, and
the second information is a second graph indicating the required time for the selected stage with respect to each predetermined period.

4. The clinical laboratory test information system according to claim 3, wherein
the controller causes the display unit to display, as the first information, a bar graph stacking the required time for each stage with respect to each predetermined period, such that each stage is selectable.

5. The clinical laboratory test information system according to claim 3 or claim 4, wherein
if a plurality of stages are selected by the operating device, the controller causes the display unit to display, as the second information, a bar graph stacking the required time for each of the selected stages at each of the predetermined period.

6. The clinical laboratory test information system according to claim 5, wherein
the memory stores a combination of a plurality of stages, and
the controller causes the display unit to show a selecting part which indicates the combination, and if the selecting part is selected by the operating device, the controller causes the display unit to display a bar graph stacking the required time for each stage contained in the combination.

7. The clinical laboratory test information system according to claim 6, wherein
the memory stores at least two combinations that include a first combination of a plurality of stages and a second combination of a plurality of stages that are different from the plurality of stages contained in the first combination, and
when the display unit is displaying a bar graph stacking the required time for each stage contained in the first combination, responsive to a switching instruction, the controller controls the display unit to show a bar graph stacking the required time for each stage contained in the second combination.

8. The clinical laboratory test information system according to any one of claims 2 to 7, wherein
if any stage in any of a plurality of periods indicated in the first information is selected by the operating device, the controller causes the display unit to display the second information.

9. The clinical laboratory test information system according to any one of claims 1 to 8, wherein
the required time for each stage is an average required time per sample.

10. The clinical laboratory test information system according to any one of claims 1 to 8, wherein
the required time for each stage is an average required time per test request.

11. The clinical laboratory test information system according to claim 9 or claim 10, wherein
the controller causes the display unit to display information indicating a maximum required time and a minimum required time among required times that have been used in calculation of the average required time.

12. The clinical laboratory test information system according to any one of claims 1 to 11, wherein
the controller receives a selection of a condition for determining a clinical-laboratory-test-relating operation to be displayed, and causes the display unit to display information indicating a temporal transition of the required time for each stage in the clinical-laboratory-test-relating operation meeting the selected condition.

13. The clinical laboratory test information system according to claim 12,
wherein
the controller receives, as the condition, a selection of a type of at least one of a testing department, a test item, and a testing apparatus.

14. The clinical laboratory test information system according to any one of claims 1 to 13 further comprising
a measurement apparatus configured to measure a sample, wherein
the plurality of stages include at least one of a measuring stage of a sample performed by the measurement apparatus and an approving stage performed by a user approving a measurement result obtained from the measurement apparatus.

15. A non-transitory storage medium which stores programs executable comprehensively by at least one processor, the programs causing the at least one processor to perform a method comprising:
storing, in a memory, time information regarding a plurality of stages included in a clinical-laboratory-test-relating operation;
causing a display unit to display, based on the time information stored in the memory, first information indicating a temporal transition of a required time for each of the plurality of stages, such that each stage is selectable;
receiving a selection of a stage by an operating device from the plurality of stages indicated in the first information; and
causing the display unit to display, based on the stored time information, second information indicating a temporal transition of the required time for the stage selected by the operating device.
